Europäisches Patentamt

European Patent Office

Office européen des brevets

(19)

(11) Numéro de publication : **0 322 289 B1**

(12) **FASCICULE DE BREVET EUROPEEN**

(45) Date de publication du fascicule du brevet :
15.04.92 Bulletin 92/16

(51) Int. Cl.⁵ : **G01N 27/416**

(21) Numéro de dépôt : **88403211.1**

(22) Date de dépôt : **16.12.88**

(54) **Procédé d'analyse en continu des ions chlorures présents dans les eaux de tête d'une colonne de distillation d'hydrocarbures et analyseur pour la mise en oeuvre de ce procédé.**

(30) Priorité : **21.12.87 FR 8717821**

(43) Date de publication de la demande :
**28.06.89 Bulletin 89/26**

(45) Mention de la délivrance du brevet :
**15.04.92 Bulletin 92/16**

(84) Etats contractants désignés :
**DE ES FR GB IT**

(56) Documents cités :
**FR-A- 2 292 977
FR-A- 2 342 496
GB-A- 1 201 850
THE OIL AND GAS JOURNAL, vol. 70, no. 29,
17 juillet 1972, pages 92-98; R. HAUSLER et al.:
"Corrosion controlled in crude units' overhead"**

(56) Documents cités :
**ERDÖL UND KOHLE, vol. 28, no. 6, juin 1975,
pages 277-282; J. ELSTER:
"Overheadkorrosion in Rohöldestillationsanlagen"**

(73) Titulaire : **TOTAL RAFFINAGE DISTRIBUTION
S.A.
84, rue de Villiers
F-92538 Levallois Perret Cédex (FR)**

(72) Inventeur : **Pauly, Jean-François
1, Impasse Hélène
F-76600 Le Havre (FR)**
Inventeur : **Roussel, Gérard
Hameau du Mahiel Bretteville du Grand Caux
F-76110 Goderville (FR)**

(74) Mandataire : **Jolly, Jean-Pierre et al
Cabinet Jolly 54, rue de Clichy
F-75009 Paris (FR)**

EP 0 322 289 B1

## Description

La présente invention consiste en un procédé d'analyse en continu des ions chlorures présents dans les eaux de tête d'une colonne de distillation d'hydrocarbures, notamment de pétroles bruts, ou de résidus de distillation atmosphérique. Cette invention concerne également un analyseur propre à assurer la mise en oeuvre de ce procédé d'analyse.

Il est connu que les ions chlorures présents dans les eaux de tête d'une colonne de distillation d'hydrocarbures, notamment de pétroles bruts, proviennent généralement de sels hydrolysables contenus initialement dans les hydrocarbures, qui s'hydrolysent pour former de l'acide chlorhydrique. Cet acide chlorydrique se condense dans les circuits de refroidissement situés en aval de la tête de tour de distillation et est à l'origine de la corrosion observée dans ces circuits. Ces sels hydrolysables se composent principalement de $MgCl_2$ et de $CaCl_2$. Pour limiter la quantité de ces sels présents dans les hydrocarbures avant distillation, il est d'usage de dessaler ces hydrocarbures par des moyens connus en soi et d'injecter de la soude, après dessalage, pour réaliser l'hydrolyse de ces sels, en les remplaçant par du chlorure de sodium qui se retrouve dans les résidus de distillation.

Cependant, il est très difficile d'ajuster la quantité de soude à injecter, car maintenir un taux d'injection de soude élevé est pénalisant, en raison des problèmes que posent de fortes teneurs en sodium dans le traitement ultérieur des résidus de distillation, particulièrement ceux des résidus atmosphériques et sous vide d'un pétrole brut.

En outre, pour neutraliser l'acide chlorhydrique résiduel présent dans les vapeurs s'échappant de la tête de tour de distillation, il est connu d'injecter au moins de l'ammoniac et/ou un inhibiteur de corrosion. Le taux d'injection d'ammoniac est lié au pH mesuré des eaux de tête, qui est maintenu au voisinage de 6.

Pour ajuster les taux d'injection de soude, ou encore d'inhibiteur de corrosion, il est nécessaire de pouvoir suivre de façon continue la teneur en chlorures des eaux condensées dans les circuits de tête de distillation et cela avec un temps de réponse le plus court possible.

Actuellement, dans le cas des eaux de tête d'une distillation atmosphérique classique, l'analyse de la teneur en ions chlorures est effectuée de façon discontinue à des intervalles de temps relativement espacés, de l'ordre d'une fois par jour. Cette analyse est typiquement une argentimétrie (ou plus rarement une ionométrie), réalisée généralement au Laboratoire de Contrôle de la Raffinerie sur un échantillon, le résultat ne parvenant à l'opérateur qu'environ 24 heures après le prélèvement. Si la mesure a révélé que la teneur en ions chlorures est supérieure à la valeur désirée, l'opérateur modifie alors les taux d'injection en soude et en inhibiteur de corrosion, mais cette action n'a pas un effet immédiat. Si l'on ajoute au délai de l'analyse le retard existant entre la modification du taux d'injection de soude après le dessaleur et son effet dans les eaux de tête, on constate que le circuit de tête d'une distillation atmosphérique classique peut se trouver pendant près de 30 heures dans des conditions de corrosion sévère.

Par des mesures ou des évaluations de la vitesse de corrosion, la Demanderesse a vérifié que, lorsque la teneur en ions chlorures des eaux de tête dépasse 10 p.p.m., on observe une accélération très nette de la corrosion des circuits en tête de distillation. La connaissance en temps réel de cette valeur (teneur en ions chlorures) est donc un paramètre important pour:

1.- optimiser les débits d'injection de soude et/ou d'inhibiteur de corrosion,

2.- augmenter la durée de vie des circuits et condenseurs de tête,

3.- valoriser le résidu atmosphérique et les fiouls, en raison du problème posé par la présence du sodium dans les fiouls.

FR-A- 2 342 496 décrit un procédé d'analyse en continu des ions chlorures présents dans les eaux usées rejetées par les installations pétrochimiques, selon lequel on prélève un échantillon à analyser, on oxyde les ions sulfures en ions sulfates et l'on mesure par ionométrie les ions chlorure.

A la connaissance de la Demanderesse, il n'existe toutefois pas de procédé, ni d'appareil permettant d'effectuer de façon satisfaisante l'analyse en continu des ions chlorures présents dans les eaux de tête de distillation. En effet, deux problèmes techniques importants n'ont pu être résolus :

– dans les eaux dont on veut mesurer la teneur en ions chlorures se trouve également du soufre, sous forme d'ions sulfures $S^{2-}$ et/ou $HS^-$. Ces derniers interfèrent dans la mesure des ions chlorures, en provoquant ainsi des erreurs ou des dérives importantes dans les résultats ; on rappelle que les eaux de tête des distillations atmosphériques de pétrole brut peuvent contenir de 0 à 400 p.p.m. d'ions sulfures $S^{2-}$ et/ou $HS^-$ selon la nature du brut ;

– pendant la marche des unités de distillation, des hydrocarbures peuvent être entraînés avec l'eau, ce qui provoque un colmatage progressif de la partie sensible de l'électrode de la cellule de mesure, dans le cas où la mesure est effectuée par ionométrie, et cette mesure devient rapidement non fiable,

La présente invention a pour but de remédier aux inconvénients rappelés ci-dessus et, à cet effet, elle a pour objet, selon un premier aspect, un procédé d'analyse en continu de la teneur en ions chlorures présents dans les eaux de tête d'une colonne de distillation d'hydrocarbures (3) selon lequel on prélève sur lesdites eaux de tête un débit d'eau à analyser, on oxyde dans les eaux prélevées les ions sulfures $S^{2-}$ ou $HS^-$ en ions sulfates, et on mesure par ionométrie la teneur ions chlorures présents, ce procédé étant caractérisé par les phases suivantes, préalablement à la mesure par ionométrie :

– on élimine dans les eaux prélevées dans les eaux de tête les hydrocarbures ainsi que les autres matières en suspension ;

– on y insuffle de l'azote pour entraîner le sulfure d'hydrogène et les hydrocarbures résiduels ;

– on acidifie ledit prélèvement.

Le procédé selon l'invention permet ainsi d'éliminer les inconvénients dûs à la présence d'hydrocarbures et d'ions sulfures $S^{2-}$ dans ledit prélèvement et, de plus, d'éviter la dérive du signal de l'électrode combinée lorsque le pH est supérieur à 7. En effet, d'une part, les ions sulfates n'interfèrent pas avec ce type d'électrode et, d'autre part, le signal de ce type d'électrode est stable lorsque le pH est maintenu entre 2 et 7,

Pour l'homme de l'art, l'opération qui consiste à insuffler de l'azote dans ledit prélèvement en vue d'entraîner le sulfure d'hydrogène et les hydrocarbures peut être faite en amont ou en aval des opérations d'oxydation et d'acidification dudit prélèvement, puisque seules varieront les quantités de sulfates provenant de l'oxydation d'un sulfure d'hydrogène,

Dans une première forme de mise en oeuvre de l'invention, on élimine les hydrocarbures du prélèvement des eaux de tête, en les filtrant sur un filtre à charbon actif,

Dans une deuxième forme de réalisation, le prélèvement d'eaux de tête peut être amené dans une capacité comprenant au moins un détecteur d'hydrocarbures et au moins un conduit d'évacuation, équipé de vanne(s) reliée(s) électriquement audit détecteur, permettant la circulation dudit prélèvement vers la cellule de mesure et, également, l'évacuation de ce prélèvement lorsque les hydrocarbures occupent un volume trop important dans ladite capacité.

Pour détecter la présence d'hydrocarbures dans ce prélèvement, il est possible d'utiliser des sondes capacitives, par exemple une sonde commercialisée sous l'appellation EFFECTOR, par la firme IFM Electronic ou tout autre moyen adéquat.

La détection d'hydrocarbures dans ladite capacité a pour avantage de pallier les aléas de fonctionnement de la régulation de niveau d'eau du ballon de tête de l'unité de distillation, qui peuvent se traduire par une évacuation trop importante et non prévisible d'hydrocarbures dans le circuit des eaux de tête de la tour de distillation à certains moments.

L'oxydation des ions sulfures $S^{2-}$ ET/OU $HS^-$ en ions sulfates est obtenue par injection dans ledit prélèvement, épuré d'une grande partie des hydrocarbures, d'une solution dite oxydante, dont le pouvoir oxydant est suffisant, au pH dudit prélèvement, pour oxyder les ions sulfures $S^{2-}$ et/ou $HS^-$ en ions sulfates, mais insuffisant pour transformer les ions chlorures en ions chlorates. Cette injection de la solution oxydante est effectuée à un pH tel qu'elle permet d'éviter la formation de soufre libre qui aurait tendance à venir colmater la partie sensible de la (ou des) électrode(s) de mesure.

Ce pouvoir oxydant est fonction des potentiels respectifs, au pH dudit prélèvement, des équilibres oxydants-réducteurs des couples ioniques en présence.

Ainsi, pour oxyder les ions sulfures en ions sulfates, la solution oxydante peut être choisie dans le groupe constitué par les bromates $BrO_3^-$, les bichromates $Cr_2O_7^{2-}$, les permanganates $MnO_4^-$, les nitrates de métaux alcalins, alcalinoterreux ou autres et de tout autre anion oxydant pour les ions sulfures, mais non oxydant pour les ions chlorures. La concentration en ions oxydants de ladite solution est comprise entre 0,1 M et 5 M et, de préférence, entre 0,5 M et 3 M.

Dans une première forme de réalisation de l'invention, cette oxydation devant être effectuée à un pH tel qu'elle permette d'éviter la formation de soufre libre, préalablement ou simultanément, un agent alcalin est ajouté, de telle manière que le pH soit supérieur à 7, par exemple une solution de soude, de concentration comprise typiquement entre 0,5 N et 2 N. Une solution oxydante préférée de cette première forme de réalisation de l'invention sera constituée d'une solution aqueuse de permanganate de potassium et de soude. Dans une deuxième forme de réalisation, il est nécessaire d'ajouter simultanément ou ultérieurement un agent acidifiant au prélèvement de façon à augmenter le pouvoir oxydant de la solution oxydante introduite. On ne sortirait pas du cadre de l'invention en introduisant en une seule étape dans le prélèvement un agent qui permette simultanément d'augmenter le pouvoir oxydant de la solution oxydante et d'acidifier ledit prélèvement. Une solution oxydante préférée de cette deuxième forme de réalisation est une solution aqueuse concentrée de bromate de sodium ($NaBrO_3$).

L'acidification ultérieure dudit prélèvement à un pH compris entre 2 et 7, en vue de stabiliser la réponse de l'électrode, est obtenue par injection d'une solution acide concentrée de 0,1 à 5 N et, de préférence, de 0,5 à 2 N, d'au moins un acide fort choisi dans le groupe constitué par l'acide sulfurique, l'acide nitrique et les acides halogénés, à l'exception de l'acide chlorhydrique.

Il est à noter que le débit d'injection de la solution oxydante est compris entre 0,001 et 0,5 fois le débit de circulation dudit prélèvement dans l'analyseur.

Un autre objet de l'invention concerne l'analyseur pour la mise en oeuvre dudit procédé, qui est caractérisé en ce qu'il comprend:

– un dispositif d'élimination d'hydrocarbures, traversé par l'eau de tête prélevée au dernier ballon de tête de distillation,

– une pompe doseuse, pneumatique ou électrique, qui reprend l'eau à la sortie du dispositif d'élimination d'hydrocarbures,

– une capacité de rétention munie, à sa base, d'une source d'azote destinée à insuffler de l'azote dans ledit prélèvement et, à son sommet, d'une membrane perméable à l'azote et aux composés volatils, notamment à l'hydrogène sulfuré $H_2S$ et, éventuellement, aux hydrocarbures volatils résiduels, qui sont entraînés par l'azote ainsi insufflé,

– deux dispositifs d'injection, respectivement, d'une première solution oxydante et d'une seconde solution acidifiante, comprenant chacun, respectivement, une pompe doseuse et un réservoir contenant ladite solution,

– une cellule de mesure, constituée d'un vase électrolytique fermé, équipé d'une électrode combinée spécifique pour la mesure de la concentration en ions chlorures dudit prélèvement traversant ledit vase, par exemple l'électrode combinée du type CE 9417 B, commercialisée par la firme ORION,

– et une électronique associée, reliée à ladite électrode, qui donne la teneur en ions chlorures présents dans l'eau, sous toute forme appropriée.

L'électrode combinée spécifique est étalonnée de façon connue en soi, à l'aide de solutions étalons, représentatives de l'eau de tête après traitement (oxydation et acidification) et contenant du chlorure d'ammonium qui a été ajouté en différentes concentrations.

Dans une première forme de réalisation du dispositif d'élimination des hydrocarbures, celui-ci peut être constitué par un filtre à charbon actif.

Dans une deuxième forme de réalisation, préférée en ce qu'elle permet de réagir de façon plus efficace aux à-coups d'hydrocarbures arrivant dans les eaux recueillies au dernier ballon de tête, le dispositif d'élimination est constitué d'une capacité équipée d'un détecteur de niveau d'hydrocarbures, de préférence d'une sonde capacitive de type commercial. Ladite capacité comprend également un premier circuit de sortie, situé dans la partie supérieure de ladite capacité, permettant l'élimination des hydrocarbures, et un second circuit, situé dans sa partie inférieure, conduisant le prélèvement d'eau sans hydrocarbures vers la cellule de mesure. Ces circuits sont avantageusement équipés de vanne(s) dont le mouvement est commandé par ledit détecteur de niveau d'hydrocarbures, en vue de couper la circulation dudit prélèvement vers la cellule de mesure, lorsque le volume d'hydrocarbures dans la capacité est trop important et qu'il y a des risques d'entraînement, par ce second circuit, vers la cellule de mesure d'hydrocarbures, susceptibles de modifier la réponse de l'électrode combinée. L'homme de l'art pourrait avantageusement supprimer la vanne située sur le second circuit de sortie de la capacité et utiliser la pompe doseuse, située en aval, pour couper le flux liquide provenant de cette capacité.

Pour être implanté dans un site industriel, tout analyseur doit présenter des garanties de sécurité; aussi l'analyseur selon l'invention doit-il satisfaire à ces exigences. Il se caractérise en ce que l'ensemble de ses éléments constitutifs sont enfermés dans un boîtier de sécurité, qui présentera, de préférence, des caractéristiques d'antidéflagrance, lorsque utilisé dans une raffinerie.

L'invention sera à présent décrite en regard des dessins annexés, donnés à titre non limitatif, dans lesquels:

La figure 1 représente un exemple d'unités de traitement du pétrole jusqu'à sa distillation et la position de l'analyseur de chlorures sur le circuit des vapeurs de tête de distillation.

La figure 2 représente le schéma de la forme préférée de l'analyseur des ions chlorures selon l'invention, et

La figure 3 représente le diagramme potentiel d'oxydo-réduction/pH, dit de POURBAIX, des zones d'existence des espèces $H_2S$, $HS^-$, $S°$, $SO_4^-$, $Cl$, et $ClO_4^-$.

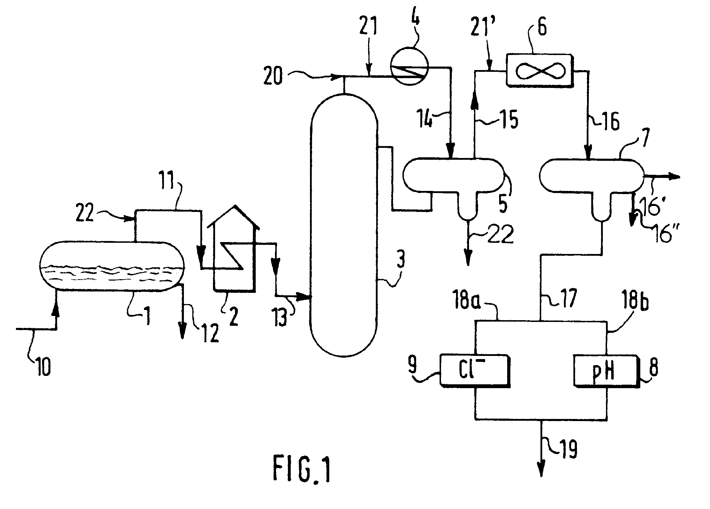

En référence à la figure 1, un circuit de traitement de pétrole brut comprend un dessaleur 1, dans lequel la plupart des sels hydrolysables du pétrole brut amené par la conduite 10 sont éliminés, puis un four de chauffe 2, vers lequel ce pétrole brut est amené par la conduite 11, la conduite 12 permettant l'élimination des eaux salines, et une colonne de distillation atmosphérique 3 du pétrole brut, amené par la conduite 13.

Les vapeurs de tête de distillation qui s'échappent au sommet de la colonne de distillation 3 passent ensuite, via la conduite 14, à travers un premier échangeur de chaleur 4, dans lequel leur température est suffisamment abaissée pour que seuls les hydrocarbures les plus lourds puissent se condenser; ils sont recueillis dans un premier ballon de tête 5, tandis que la vapeur d'eau débarrassée des hydrocarbures les plus lourds est amenée à traverser un dispositif aéroréfrigérant 6 via la conduite 15.

Les hydrocarbures les plus lourds sont recueillis en 22 à la base du premier ballon de tête 5. L'eau condensée, évacuée du dispositif aéroréfrigérant 6 par la conduite 16, est récupérée à la base d'un deuxième ballon de tête 7 via la conduite 17. Les gaz incondensables s'échappent de ce dernier par un conduit 16′ et les hydrocarbures par un conduit 16″. La conduite 17 se sépare en deux circuits parallèles, le circuit 18a, sur lequel est branché l'analyseur de chlorures 9 selon l'invention, et le circuit 18b, sur lequel on pratique des mesures du pH en continu au moyen d'un pH-mètre 8; les deux circuits se rejoignent dans la conduite 19, les eaux condensées étant envoyées vers une unité de traitement en aval.

Afin de limiter la corrosion dans tout ce circuit de tête de distillation, une injection d'ammoniac NH₃ est effectuée en 20, pour maintenir le pH dans l'eau condensée en 7 à une valeur d'environ 6. Cette injection est asservie à la mesure du pH donnée par l'analyseur 8 de pH. Une première injection d'inhibiteur de corrosion, en 21, et éventuellement une deuxiéme, en 21', permettent de limiter la corrosion par les premières gouttes de rosée qui se forment au niveau du dispositif aéroréfrigérant 6 et qui contiennent une très forte concentration d'acide chlorhydrique, de l'ordre de quelques centaines de p.p.m. Ces injections viennent en complément d'une injection de soude réalisée en 22 sur la conduite 11 , pour lutter efficacement contre la corrosion.

La surveillance en continu, par l'analyseur 9, de la teneur en chlorure des eaux condensées de tête permet d'agir en continu, rapidement et de façon précise, sur les taux d'injection en 21 et éventuellement 21' de l'inhibiteur de corrosion et, également, sur l'injection de soude en 22.

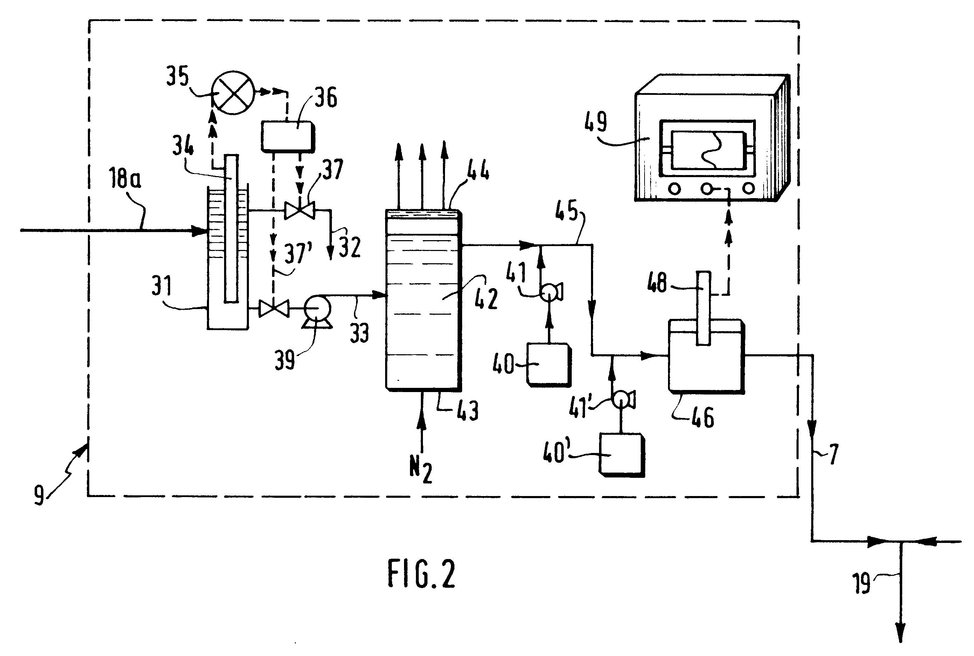

On décrira à présent l'analyseur des chlorures 9, selon l'invention, représenté sur la figure 2.

Cet analyseur 9 comprend une capacité 31, dans laquelle est amené le prélèvement qui arrive par la conduite 18a. Elle comprend deux conduites de sortie, dont l'une, 32, dans sa partie supérieure, permet l'évacuation du prélèvement surchargé en hydrocarbures et la seconde, 33, dans la partie inférieure de la capacité 31, facilite la conduite du prélèvement vers la suite de l'analyseur 9.

La capacité 31 est munie, à son sommet, d'une sonde capacitive 34 de type commercial (par exemple une sonde EFFECTOR), sensible aux hydrocarbures, reliée au détecteur 35, qui, selon le signal provenant de la sonde 34, va agir par l'intermédiaire du relais 36 sur le mouvement des vannes, respectivement 37, située sur la conduite 32, et 37', située sur la conduite 33.

Lorsque la sonde 34 détecte des hydrocarbures dans le fond de la capacité 31, les vannes 37 et 37' sont actionnées de façon à fermer l'accès à la suite de l'analyseur et à évacuer le prélèvement surchargé en hydrocarbures par la conduite d'évacuation 32. Les vannes 37 et 37' reviennent à leur position initiale lorsque la sonde 34 ne détecte plus d'hydrocarbures au fond de la capacité 31. L'eau sort alors par la conduite 33 et traverse une pompe doseuse 39, pneu-matique ou électrique. Après la pompe 39, l'eau épurée est amenée dans la capacité de rétention 42. A la base 43 de cette capacité, on insuffle de l'azote à un débit prédéterminé, pour entraîner vers la membrane poreuse 44 placée au sommet de la capacité 42 les hydrocarbures volatils résiduels et l'hydrogène sulfuré. La solution oxydante et la solution acidifiante, stockées respectivement dans les réservoirs 40 et 40', sont pompées par deux pompes doseuses, respectivement, 41 et 41', (ou éventuellement une seule pompe doseuse à deux têtes), puis injectées dans la conduite 45, qui véhicule l'eau insufflée sortant de la capacité de rétention 42. Ainsi qu'il sera expliqué ci-après, l'injection de la solution acidifiante est effectuée postérieurement à l'injection de la solution oxydante.

L'eau ainsi traitée est amenée, via la conduite 45, au vase électrolytique fermé, de la cellule de mesure 46, comprenant un orifice de sortie de l'eau 47, qui rejoint la conduite 19, telle que représentée à la Figure 1, et munie d'une électrode combinée spécifique 48, par exemple du type CE 9417 B, commercialisée par la Société ORION. Cette électrode est reliée à une électronique associée 49, qui traite le signal électrique généré par la cellule de mesure et le restitue sous la forme d'une information directement exploitable (affichage de la teneur en chlorures...), et/ou éventuellement enregistrable. La teneur en chlorures, qui est affichée en continu, permet à un opérateur, ou par un système d'asservissement, de modifier le taux d'injection de l'inhibiteur de corrosion et celui de la soude.

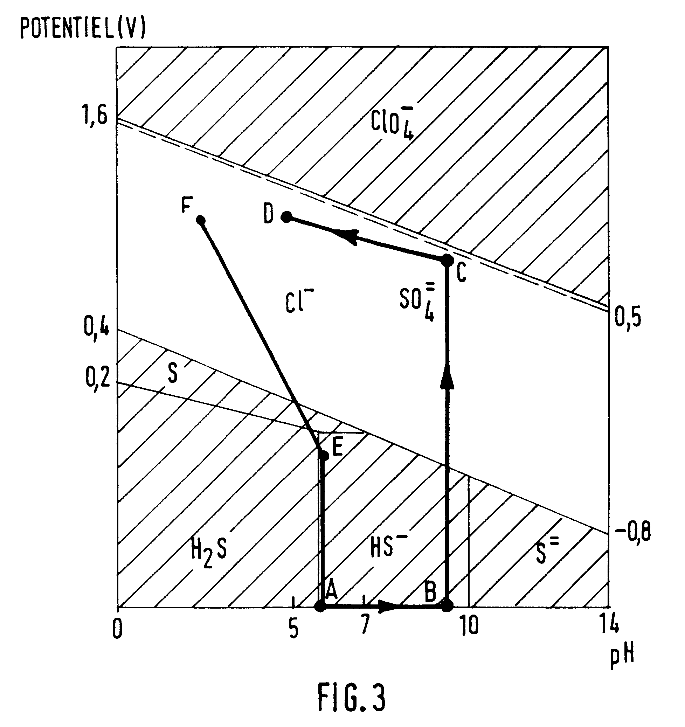

On a représenté à la Figure 3, le diagramme d'existence des espèces chlorées (Cl⁻, ClO₄⁻) et sulfurées (S°, H₂S, HS⁻, S⁻ et SO₄⁻) en fonction du potentiel d'oxydo- réduction et du pH, selon la représentation dite de POURBAIX. En abcisses, se trouve le pH, qui varie de 0 à 14, en ordonnées, le potentiel, qui varie de -1 à +2 Volts;

Les courbes en trait plein, qui séparent les domaines d'existence, sont les courbes dites de molarité, sur lesquelles l'espèce indiquée est à une concentration de 1M. Sa concentration variant exponentiellement en fonction du potentiel autour de ses courbes, on peut toutefois admettre que l'espèce n'existe plus au-delà de ladite courbe.

Un premier exemple de mise en application du procédé selon l'invention consiste, partant d'une eau de tête de pH d'environ 6 et de potentiel réducteur, représenté symboliquement par le point A sur ce diagramme, à se déplacer jusqu'au point B, de pH d'environ 9, en ajoutant un agent alcalin, par exemple de la soude, puis à se déplacer jusqu'en C, de pH d'environ 9 et de potentiel supérieur à 0,4 volt, en ajoutant un agent oxydant, par exemple du permanganate de potassium, puis à revenir jusqu'en D, de pH d'environ 5 et de potentiel supérieur à 1,1 volt, en ajoutant un agent acidifiant, par exemple de l'acide nitrique.

En fait, avec l'analyseur tel que décrit ci-dessus, le déplacement s'effectue directement de A en C, au moyen de l'addition d'une solution simultanément alcaline et oxydante; l'homme de l'art serait à même d'imaginer un analyseur comportant trois dispositifs d'injection, dont un premier pour l'injection d'un agent alcalin, sans pour cela faire oeuvre inventive, donc sans sortir du cadre de la présente invention.

L'intérêt de ce déplacement dans le susdit diagramme de POURBAIX consiste principalement à éviter le domaine d'existence de l'espèce soufre libre (S°), qui, une fois formée, est stable et colmate la partie sensible de l'électrode, réalisée en fritté d'argent dans ce type d'électrode combinée.

Un second exemple de mise en application du procédé selon la présente invention consiste, partant de la même eau de tête que dans l'exemple précédent, à pH = 6 et de potentiel réducteur, représentée par le même point A sur ce diagramme, à se déplacer jusqu'au point E de potentiel inférieur à - 0,2 Volt en ajoutant un agent oxydant tel que du bromate de sodium; puis à se déplacer jusqu'au point F de ph 2 et de potentiel supérieur à 0,4 Volt, en ajoutant un agent acidifiant, par exemple de l'acide nitrique. La présence d'acide nitrique dans le prélèvement augmente le pouvoir oxydant des ions bromates, ce qui permet d'éviter la formation de soufre libre lors de la traversée de la zone de stabilisation du soufre libre (S°).

Le procédé d'analyse en continu et l'analyseur selon l'invention permettent donc de limiter la corrosion des circuits et condenseurs de tête et, de ce fait, d'augmenter leur durée de vie. Ils permettent également de réaliser des économies importantes sur les quantités d'inhibiteurs injectées dans les eaux de tête, et d'éviter les inconvénients d'une surinjection de soude dans le pétrole brut.

**Revendications**

1. Procédé d'analyse en continu de la teneur en ions chlorures présents dans les eaux de tête d'une colonne de distillation d'hydrocarbures (3) selon lequel on prélève sur lesdites eaux de tête un débit d'eau à analyser, on oxyde dans les eaux prélevées les ions sulfures $S^{2-}$ ou $HS^-$ en ions sulfates, et on mesure par ionométrie la teneur ions chlorures présents, ce procédé étant caractérisé par les phases suivantes, préalablement à la mesure par ionométrie :
   – on élimine dans les eaux prélevées dans les eaux de tête les hydrocarbures ainsi que les autres matières en suspension ;
   – on y insuffle de l'azote pour entraîner le sulfure d'hydrogène et les hydrocarbures résiduels ;
   – on acidifie ledit prélèvement.

2. Procédé selon la revendication 1, caractérise en ce que l'élimination des hydrocarbures et des matières en suspension dans ledit prélèvement est effectuée par filtration au travers d'un filtre à charbon actif.

3. Procédé selon la revendication 1, caractérisé en ce que l'élimination des hydrocarbures dans ledit prélèvement est obtenue par décantation dans une capacité (31), comprenant au moins un détecteur d'hydrocarbures (34) et au moins un conduit d'évacuation (32), équipé de vanne(s) reliée(s) électriquement audit détecteur (34), permettant la circulation dudit prélèvement vers la cellule de mesure et, également, l'évacuation de ce prélèvement lorsque les hydrocarbures occupent un volume trop important dans ladite capacité (31).

4. Procédè selon l'une des revendications 1 à 3, caractérisé en ce que les ions sulfures $S^{--}$ et/ou $HS^-$ sont oxydes en ions sulfates par injection dans ledit prélèvement d'une solution oxydante, dont le pouvoir oxydant est suffisant pour oxyder les ions sulfures en ions sulfates au pH dudit prélèvement, mais insuffisant pour oxyder les ions chlorures en ions chlorates, cette injection étant effectuée à un pH tel qu'elle permet d'éviter la formation de soufre libre.

5. Procédé selon l'une des revendications 1 à 4, caractérisé en ce que la solution alcaline-oxydante contient au moins un sel choisi dans le groupe constitué par les bromates $BrO_3^-$, les chromates $Cr_2O_7^{2-}$, les permanganates $MnO_4^-$, les nitrates $NO_3^-$ de métaux alcalins, alcalinoterreux ou autres et de tout autre anion oxydant pour les ions sulfures, mais non oxydant pour les ions chlorures, et éventuellement un agent alcalin.

6. Procédé selon la revendication 5, caractérisé en ce que la solution oxydante est une solution aqueuse concentrée de 0,1 M à 5 M et, de préférence, de 0,5 M à 2M, de permanganate de potassium $KMnO_4$, et de 0,5 à 2 N de soude.

7. Procédé selon la revendication 5, caractérisé en ce que la solution oxydante est une solution aqueuse concentrée de 0,1 M à 5 M et, de préférence, de 0,5M à 2M de bromate de sodium $NaBrO_3$.

8. Procédé selon l'une des revendications 1 à 7, caractérisé en ce que ledit prélèvement est acidifié ultérieurement par injection d'une solution acidifiante, concentrée de 0,1N à 5N et de 0,5N à 2N, d'au moins un acide fort choisi dans le groupe constitué par l'acide sulfurique, l'acide nitrique et les acides halogénés, exception faite de l'acide chlorhydrique.

9. Procédé selon l'une des revendications 1 à 8, caractérisé en ce que le débit d'injection de la solution oxydante est compris entre 0,001 et 0,5 fois le débit de circulation dudit prélèvement dans l'analyseur.

10. Analyseur pour la mise en oeuvre du procédé selon l'une des revendications précédentes, caractérisé en ce qu'il comprend:
   – un dispositif d'élimination des hydrocarbures (31), traversé par l'eau de tête prélevée au dernier ballon de tête de distillation,
   – une pompe doseuse (39), pneumatique ou électrique, qui reprend l'eau à la sortie du dispositif d'élimination d'hydrocarbures (31),
   – une capacité de rétention (42) munie, à sa base (43), d'une source d'azote destinée à insuffler de l'azote dans ledit prélèvement et, à son sommet, d'une membrane (44) perméable à l'azote et aux composés volatils, qui sont entraînés par l'azote ainsi insufflé,
   – deux dispositifs d'injection, respectivement, d'une première solution oxydante et d'une seconde solution acidifiante dans la conduite de circulation (45), comprenant chacun, respectivement, une pompe doseuse (41 et 41') et un réservoir (40 et 40') contenant ladite solution,
– une cellule de mesure (46), constituée d'un vase électrolytique (47) fermé, équipé d'une électrode combinée spécifique (48) pour la mesure de la concentration en ions chlorures dudit prélèvement traversant ladite cellule,
– et une électronique associée (49), reliée à ladite électrode combinée (48) qui traite le signal électrique généré par ladite électrode combinée et le restitue sous la forme d'une information exploitable et/ou éventuellement enregistrable.

11. Analyseur selon la revendication 10, caractérisé en ce que le dispositif d'élimination des hydrocarbures est un filtre à charbon actif.

12. Analyseur selon la revendication 10, caractérisé en ce que le dispositif d'élimination des hydrocarbures (31) est constitué par une capacité équipée d'un détecteur de niveau d'hydrocarbures (34), comprenant:
    – un premier circuit de sortie (32), muni d'une première vanne (37), commandée par le détecteur (34), permettant l'élimination du prélèvement lorsque les hydrocarbures occupent un volume trop important,
    – et un second circuit (33), dans la partie inférieure conduisant le prélèvement d'eau sans hydrocarbures vers la cellule de mesure (46), ce second circuit (33) étant muni d'une seconde vanne (37') dont le mouvement est commandé simultanément par ledit détecteur (34), en vue de couper la circulation dudit prélèvement vers ladite cellule (46) lorsque les hydrocarbures occupent un volume trop important.

13. Analyseur selon l'une des revendications 10 à 12, caractérisé en ce que l'ensemble des éléments de l'analyseur (9) est enfermé dans un boîtier de sécurité, répondant aux normes de sécurité de l'industrie utilisatrice.

## Patentansprüche

1. Kontinuierliches Analyseverfahren des Gehaltes der in dem Kopfwasser einer Destillierkolonne für Kohlenwasserstoff anwesenden chlorierten Ionen, bei dem von besagtem Kopfwasser eine Wassermenge zum Analysieren angezapft wird, die Sulfidionen $S^{2-}$ oder $HS^-$ im angezapften Wasser zu Sulfationen oxidiert werden, und durch Ionometrie der Gehalt an vorhandenen chlorierten Ionen gemessen wird, und die:es Verfahren durch die folgenden Verfahrensschritte, vor dem Messen durch Ionometrie, gekennzeichnet sind:
    – man beseitigt im angezapften Wasser im Kopfwasser die Kohlenwasserstoffe sowie die anderen in Suspension befindlichen Stoffe;
    – man bläst dort Stitkstoff hinein, um Schwefelwasserstoff und die übrigen Kohlenwasserstoffe mitzunehmen;
    – man säuert besagte Anzapfung an.

2. Verfahren gemäß Anspruch 1, dadurch gekennzeichnet, daß das Beseitigen der Kohlenwasserstoffe und der in Suspension befindlichen Stoffe in besagter Anzapfung durch Querfiltration eines Aktivkohlefilters bewirkt wird.

3. Verfahren gemäß Anspruch 1, dadurch gekennzeichnet, daß das Beseitigen der Kohlenwasserstotfe in besagter Anzapfung durch Dekantation in einer Kapazität (31) erreicht wird, die mindestens ein Kohlwasserstoffdetektor (34) und mindestens einen Entleerungskanal (31) enthält, ausgestattet mit einem oder mehreren elektrischen Schaltschiebern an besagtem Detektor (34), wobei die Zirkulation der besagten Anzapfung gegen die Meßzelle gestattet wird, und auf gleiche Art das Entleeren von dieser Anzapfung, wenn die Kohlenwasserstoffe ein zu bedeutendes Volumen in besagter Kapazität (31) einnehmen.

4. Verfahren gemäß einem der Ansprüche 1 bis 3, dadurch gekennzeichnet, daß die Sulfidionen $S^-$ und/oder $HS^-$ in Sulfationen durch Injizieren einer Oxidationslösung in besagte Anzapfung oxidiert werden, deren Oxidationskraft ausreicht, um die Sulfidionien in Sulfationen des pH-Wertes besagter Anzapfung zu oxidieren, aber nicht dazu ausreicht, chlorierte Ionen in Chlorationen zu oxidieren, wobei diese Injektion bei einem pH-Wert. ausgeführt wird, der so beschaffen ist, daß die Ausbildung von freiem Schwefel verhindert wird.

5. Verfahren gemäß einem der Ansprüche 1 bis 4, dadurch gekennzeichnet, daß die alkalisch - oxidierende Lösung mindestens ein Salz enthält, dasa aus einer Gruppe ausgesucht ist, die gebildet wird durch die Bromate $BrO_3^-$, die Chromate $Cr_2O_7^{2-}$, die Permanganate $MnO_4^-$, die Nitrate $NO_3^-$ von alkalischen, erdalkalischen oder anderen Metallen, und vom ganz anderen Anion oxidierend für die Sulfidionen, aber nicht oxidierend für die chlorierten Ionen, und evtl. einem alkalischen Mittel.

6. Verfahren gemäß Anspruch 5, dadurch gekennzeichnet, daß die oxidierende Lösung eine wässrige Lösung ist mit einer Konzentration von 0,1 M bis 5 M und von 0,5 M bis 2 M des Kaliumpermanganat $KMnO_4$, und von 0,5 bis 2 N von Soda.

7. Verfahren gemäß Anspruch 5, dadurch gekennzeichnet, daß die oxidierende Lösung eine wässrige Lösung ist mit einer Konzentration von 0,1 M bis 5 M und von 0,5 M bis 2 M des Natriumbromat $NaBrO_3$.

8. Verfahren gemäß einem der Ansprüche 1 bis 7, dadurch gekennzeichnet, daß besagte Anzapfung außerdem durch Injektion einer sauren Lösung angesäuert wird, die eine Konzentration von 0,1 N bis 5 N und 0,5 N bis 2 N mindestens einer starken Säure aufweist, die aus einer Gruppe ausgewählt wurde, die aus der Schwefelsäure, der Sapetersäure und den Halogensäuren besteht, mit Ausnahme von Chlorwasserstoffsäure.

9. Verfahren gemäß einem der Ansprüche 1 bis 8, dadurch gekennzeichnet, daß die Injektionsmenge der oxidierenden Lösung zwischen 0,001 und 0,5 mal der Zirkulationsmenge besagter Anzapfung in der Analysevorrichtung enthalten ist.

10. Analysevorrichtung zur Verwendung in dem Verfahren gemäß einem der vorhergehenden Ansprüche, dadurch gekennzeichnet, daß es enthält:
– eine Einrichtung zum Beseitigen der Kohlenwasserstoffe (31), die das angezapfte Kopfwasser am letzten Ballon des Destillationskopfes kreuzt,
– eine Dosierpumpe (39), die pneumatisch oder elektrisch ist und das Wasser am Ausgang der Einrichtung zum Beseitigen der Kohlenwasserstoffe zurückbefördert,
– eine Rückhaltekapazität (42), die an ihrem Boden (43) mit einer Stickstoffquelle versehen ist, die dazu bestimmt ist, Stickstoff in besagte Anzapfung einzublasen und an ihrer Spitze mit einer Membran (44), die für Stickstoff und flüchtige Verbindungen durchlässig ist, die, durch den Stickstoff mitgerissen, ebenso eingeblasen wurden,
– zwei Injektionseinrichtungen, einer ersten oxidierenden Lösung und bzw. einer zweiten angesäuerten Lösung in der Zirkulationsleitung (45), wobei jede jeweils eine Dosierpumpe (41 und 41′) und ein Reservoir (40 und 40′) enthält, das besagte Lösung enthält,
– eine Meßzelle (46), bestehend aus einem geschlossenen elektrolytischen Gefäß (43), ausgestartet mit einer kombinierten spezifischen Elektrode (48) zum Messen der Konzentration an chlorierten Ionen der besagten Anzapfung, die besagte Zelle durchquert,
– und ein elektronisthes Element (49), das mit besagter kombinierter Electrode (48) verbunden ist und das elektrische Signal behandelt, das durch besagte kombinierte Elektrode erzeugt wird und es in einer Form von Information wieder herstellt, die genutzt werden kann und/oder evtl. aufzeichenbar ist.

11. Analysevorrichtung gemäß Anspruch 10, dadurch gekennzeichnet, daß die Einrichtung zum Beseitigen der Kohlenwasserstoffe ein Filter aus Aktivkohle ist.

12. Analysevorrichtung gemäß Anspruch 10, dadurch gekennzeichnet, daß die Einrichtung zum Beseitigen der Kohlenwasserstoffe (31) aus einer Kapazität gebildet wird, die mit einem Detektor für das Niveau der Kohlenwasserstoffe ausgestattet ist, und umfaßt:

– einen ersten Ausgangskreislauf (32), der mit einem ersten Ventil (37) versehen ist, das durch den Detektor (34) gesteuert wird, wobei das Entfernen der Anzapfung gestattet wird, wenn die Kohlenwasserstoffe ein zu bedeutendes Volumen einnehmen,
– und einen zweiten Kreislauf (33) in einem unteren Abschnitt, der die Anzapfung des Wassers ohne Kohlenwasserstoffe zur Meßzelle (46) leitet, wobei dieser zweite Kreislauf (33) mit einem zweiten Ventil (37′) ausgestattet ist, dessen Bewegung simultan durch besagten Detektor (34) gesteuert wird, um die Zirkulation besagter Anzapfung zur besagten Zelle (46) zu unterbrechen, wenn die Kohlenwasserstoffe ein zu bedeutendes Volumen einnehmen.

13. Analysevorrithtung gemäß einer der Ansprüche 10 bis 12, dadurch gekennzeichnet, daß die Anordnung der Elemente der Analysevorrichtung (9) in einem Sicherheitsgehäuse eingeschlossen ist, entsprechend den Sicherheitsnormen der verwendenden Industrie.

## Claims

1. A process for the continuous analysis of the content of chloride ions present in the first-fraction waters of a hydrocarbon distillation column (3), in accordance with which a sample flow of water to be analysed is taken from said first-fraction waters, the $S^{2-}$ or $HS^-$ sulphide ions in the samples of water taken are oxidised to sulphate ions, and the content of chloride ions present are measured by ionometry, said process being characterised by the following stages, prior to the measurement by ionometry:
– the hydrocarbons and other substances in suspension are eliminated from the samples of water taken from first-fraction waters;
– nitrognen is blown in so as to entrain the hydrogen sulphide and residual hydrocarbons;
– said sample is acidified.

2. A process according to claim 1, characterised in that the elimination of the hydrocarbons and the substances in suspension from said sample is effected by filtration through an activated carbon filter.

3. A process according to claim 1, characterised in that the elimination of the hydrocarbons from said sample is carried out by decanting in a vessel (31) comprising at least one hydrocarbons detector (34) and at least one outlet duct (32) provided with a valve or valves connected electrically to said detector (34), allowing the circulation of said sample towards a measuring cell and also the discharge of said sample when the hydrocarbons occupy an excessively large volume of said vessel (31).

4. A process according to any one of claims 1 to 3, characterised in that the $S^-$ and/or $HS^-$ sulphide ions are oxidised to sulphate ions by injecting into said samples an oxidising solution whose oxidising capacity is sufficient to oxidise the sulphide ions to sulphate ions at the pH value of said sample but insufficient to oxidise the chloride ions to chlorate ions, said injection being carried out at a pH value such that it enables the formation of free sulphur to be avoided.

5. A process according to any one of claims 1 to 4, characterised in that the oxidising alkaline solution contains at least one salt chosen from the group comprising the $BrO_3^-$ bromates, the $Cr_2O_7^{2-}$ chromates, the $MnO_4^-$ permanganates, the $NO_3^-$ nitrates of alkaline, alkaline earth or other metals and any other anion which is oxidising for the sulphide ions but not oxidising for the chloride ions, and optionally an alkaline agent.

6. A process according to claim 5, characterised in that the oxidising solution is a concentrated aqueous solution of 0.1M to 5m and 0.5M to 2M of potassium permanganate $KMnO_4$ and 0.5 to 2N of soda.

7. A process according to claim 5, characterised in that the oxidising solution is a concentrated aqueous solution of 0.1M to 5M and 0.5M to 2M of sodium bromate $NABrO_3$.

8. A process according to any one of claims 1 to 7, characterised in that said sample is subsequently acidified by injecting a concentrated acidifying solution of 0.1N to 5N and 0.5N to 2N of at least one strong acid chosen from the group comprising sulphuric acid, nitric acid and the halogenated acids, with the exception of hydrochloric acid.

9. A process according to any one of claims 1 to 8, characterised in that the rate of injection of the oxidising solution is between 0.001 and 0.5 times the rate of circulation of said sample in the analyser.

10. An analyser for carrying out the process according to any one of the preceding claims, characterised in that it comprises:
– a device (31) for eliminating hydrocarbons, through which passes the first-fraction water sampled at the last first-fraction flask;
– a pneumatic or electric metering pump (39) which takes the water from the outlet of the device (31) for eliminating hydrocarbons;
– a reservoir (42) provided at its bottom (43) with a nitrogen supply intended to blow nitrogen into said sample and, at its top, with a membrane (44) which is permeable to nitrogen and the volatile compounds which are entrained by the nitrogen thus blown in;
– two devices for injecting, respectively, a first oxidising solution and a second acidifying solution into the circulation duct (45), each comprising respectively a metering pump (41 and 41′) and a reservoir (40 and 40′) containing said solution;
– a measuring cell (46) comprising a closed electrolytic vessel (47) provided with a specific combined electrode (48) for measuring the concentration of chloride ions of said sample passing through said cell;
– and a associatied electronic unit (49) which is connected to said combined electrode (48) and which processes the electric signal generated by said combined electrode and restores it in the form of data which can be utilised and/or optionally recorded.

11. An analyser according to claim 10, characterised in that the device for eliminating hydrocarbons is an activated carbon filter.

12. An analyser according to claim 10, characterised in that the device (31) for eliminating hydrocarbons is composed of a vessel provided with a hydrocarbon level detector (34) comprising:
– a first output circuit (32) provided with a first valve (37) controlled by the detector (34) enabling the sample to be eliminated when the hydrocarbons occupy an excessively large volume;
– and a second circuit (33) in the lower portion conveying the sample of water without hydrocarbons towards the measuring cell (46), said second circuit (33) being provided with a second valve (37′), the movement of which is controlled simultaneously by said detector (34) for the purpose of cutting off the circulation of said sample towards said cell (46) when the hydrocarbons occupy an excessively large volume.

13. An analyser according to any one of claims 10 to 12, characterised in that the assembly of the elements of the analyser (9) is enclosed in a safety housing which complies with the safety standards of the user industry.

FIG.1

FIG.2

EP 0 322 289 B1

FIG.3